# FASCICULE DE BREVET EUROPEEN

(11) **EP 0 358 556 B1**
(45) Date de publication et mention de la délivrance du brevet: **11.05.1994**
(21) Numéro de dépôt: 89402399.3
(22) Date de dépôt: 04.09.1989
(51) Int. Cl.: B01D 57/02, C07K 3/14, G01N 27/26

(54) **Procédé et dispositif d'électrophorèse multiple pour assurer la migration contrôlée de macromolécules dans des plaques de gel**
Verfahren und Vorrichtung zur multiplen Elektrophorese mit kontrollierter Wanderung von Makromolekülen in Gelplatten
Method and apparatus for multiple electrophoresis ensuring controlled migration of macromolecules in layers of gel

(30) Priorité: 06.09.1988 FR 8811626; 23.11.1988 FR 8815246
(43) Date de publication de la demande: 14.03.1990
(73) Titulaire: BERTIN & CIE, F-78373 Plaisir Cédex (FR)
(72) Inventeur: Dutertre, Bernard, F-92200 Neuilly-Sur-Seine (FR)
(74) Mandataire: Ramey, Daniel

(56) Documents cités:
- EP-A- 234 836
- EP-A- 0 256 737
- WO-A-84/02001
- WO-A-87/02132
- DE-A- 2 107 092
- DE-A- 2 204 697
- US-A- 3 879 280
- US-A- 4 416 761

## Description

L'invention concerne un procédé et un dispositif d'électrophorèse multiple, permettant d'assurer la migration contrôlée de macromolécules dans des plaques de gel.

On utilise actuellement des techniques de séparation de macromolécules, telles que des protéines ou des acides nucléiques, par électrophorèse au moyen d'un champ électrique appliqué aux extrémités longitudinales d'une plaque rectangulaire d'un gel approprié, par exemple d'agarose ou de polyacrylamide. Des échantillons de macromolécules, par exemple d'acides nucléiques, à séparer sont déposés dans des puits formés dans le gel, le long d'un bord de la plaque, puis l'ensemble est immergé dans un liquide approprié d'électrophorèse. Les électrodes placées contre le bord précité de la plaque et le bord opposé de celle-ci sont reliées à des potentiels différents, de sorte qu'un champ électrique s'établit entre les électrodes dans un sens correspondant à la direction souhaitée de migration des macromolécules dans la plaque de gel. Sous l'effet du champ électrique, les macromolécules des échantillons logés dans les puits se déplacent vers le bord opposé de la plaque, à travers le gel, à des vitesses qui sont fonction notamment de leur masse moléculaire, de sorte qu'au bout d'un temps donné, des macromolécules de masses moléculaires différentes ont parcouru dans le gel des distances différentes.

Dans une technique connue, les macromolécules ainsi séparées sont ensuite transférées, soit par aspiration, soit au moyen d'un champ électrique orienté perpendiculairement à la plaque dans le sens voulu, sur une membrane placée sur une grande face de la plaque de gel, en vue de leur hybridation et de leur détection ultérieures, cf. par example WO-A-87/02132.

Cette technique et les appareils qui ont été réalisés pour sa mise en oeuvre, relèvent essentiellement du laboratoire, c'est-à-dire qu'il s'agit d'appareils de petite taille, fonctionnant à faible cadence, traitant les plaques de gel l'une après l'autre, etc...

En outre, les séparations réalisées avec ces appareils ne sont pas parfaitement répétitives et peuvent varier d'une plaque à l'autre ou d'un échantillon à l'autre dans la même plaque, ne serait-ce que parce que le champ électrique développé entre les deux électrodes n'est pas uniforme, en raison de l'hétérogénéité du milieu traversé, de sorte que des macromolécules identiques, de même masse moléculaire, peuvent migrer sur des distances différentes si elles sont placées en des points différents d'une même plaque, ou sur des plaques différentes.

Il en résulte notamment des difficultés d'interprétation des résultats, et une impossibilité d'automatiser les appareils de séparation de macromolécules par électrophorèse.

Le document DE-A-2 107092 décrit une cuve d'électrophorèse dans laquelle un boîtier comprenant des plaques de gel verticales est disposé entre des électrodes parallèles entre elles et aux plaques et qui s'étendent dans deux plans horizontaux au-dessus et au-dessous du boîtier. Ce dispositif connu permet la migration de macromolécules dans une direction dans les plaques de gel et impose que les plaques de gel soient fabriquées dans le boîtier.

Le document WO-A-84/02001 décrit un dispositif d'électrophorèse pour la séparation de macromolécules dans des plaques de gel empilées horizontalement, au moyen d'électrodes verticales qui sont réparties régulièrement autour de la pile de plaques, le long des côtés des plaques. Ce dispositif connu ne permet que la migration des macromolécules dans des plans parallèles aux plaques de gel.

Le document EP-A-0234836 décrit une cuve d'électrophorèse dans laquelle une plaque de gel peut être disposée verticalement entre deux groupes d'électrodes allongées horizontales agencées de part et d'autre de la plaque de gel, dans des plans obliques par rapport à cette plaque. Pour réaliser successivement la séparation des macromolécules dans la plaque de gel, puis leur transfert sur une membrane, il faut disposer des électrodes de séparation dans la cuve, puis les retirer et disposer ensuite des électrodes de transfert dans la cuve. Entre la séparation et le transfert, la plaque de gel doit être retirée de la cuve, traitée, associée à une membrane et replacée avec la membrane dans la cuve.

L'invention a notamment pour but d'éviter les inconvénients de la technique antérieure.

Elle a pour objet un procédé et un dispositif d'électrophorèse multiple, permettant de réaliser une séparation de macromolécules dans des plaques de gel, de façon fiable, fidèle, répétitive et parfaitement automatisable.

L'invention a encore pour objet un procédé et un dispositif de ce type qui permettent de traiter simultanément un grand nombre de plaques de gel.

L'invention a encore pour objet un procédé et un dispositif du type précité, permettant de modifier à volonté les conditions de séparation des macromolécules, notamment par variation contrôlée du champ électrique appliqué aux plaques de gel pour la migration des macromolécules.

Elle propose un procédé d'électrophorèse multiple pour la migration contrôlée de macromolécules dans des plaques de gel, consistant à immerger des plaques de gel dans une cuve contenant un liquide d'électrophorèse, ces plaques comprenant des macromolécules le long d'un de leurs bords et formant au moins une pile dans laquelle elles sont parallèles et espacées les unes des autres, et à appliquer des potentiels électriques à des électrodes allongées parallèles entre elles et aux plaques de gel, immergées dans ledit liquide, et disposées le long de deux extrémités opposées de la pile de plaques dans deux plans perpendiculaires aux plaques, les électrodes d'un même plan étants portées à un même potentiel, pour créer entre ces plans un champ électrique de migration des macromolécules dans les plaques de gel, caractérisé en ce que :
- ladite cuve comprend d'autres électrodes allongées parallèles aux électrodes précitées et disposées entre les extrémités de la pile de plaques, de sorte que toutes les électrodes sont situées aux intersections de deux séries de plans, les plans d'une première série étant perpendiculaires aux plaques et à une première direction de migration des macromolécules, les plans de la seconde série étant parallèles aux plaques,
- et en ce que le procédé consiste à appliquer des potentiels électriques aux électrodes pour créer dans les plaques de gel et dans le liquide d'électrophorèse, successivement un premier champ électrique (E1) dont la direction est en tous points sensiblement parallèle à la première direction de migration et ensuite un second champ électrique (E2) parallèle à une seconde direction de migration qui est perpendiculaire aux plaques de gel,
- le premier champ électrique (E1) étant créé par application de potentiels électriques différents à des plans d'électrodes différents de la première série, les électrodes contenues dans un même plan de la première série étant à un même potentiel électrique,
- le second champ électrique (E2) étant créé par application de potentiels électriques différents à des plans d'électrodes différents de la seconde série, les électrodes contenues dans un même plan de la seconde série étant à un même potentiel électrique.

Les électrodes contenues dans des plans perpendiculaires à la direction souhaitée de migration des macromolécules définissent des surfaces équipotentielles qui sont sensiblement planes, au moins en première approximation, puisqu'elles sont définies par des droites parallèles et coplanaires.

Le champ électrique étant nécessairement normal aux surfaces équipotentielles, qui sont elles-mêmes perpendiculaires à la direction souhaitée de migration, on obtient ainsi nécessairement, dans les plaques de gel, un champ électrique orienté dans la direction voulue, au moins au niveau de chaque électrode, et cela malgré l'hétérogénéité du milieu soumis au champ électrique.

Il en résulte un contrôle de l'amplitude et de l'orientation du champ électrique à travers les plaques de gel, qui est tel que l'on obtient, dans des conditions identiques, des séparations de macromolécules et des mesures parfaitement répétitives.

Il en découle la possibilité d'automatiser les processus de séparation de macromolécules, et de les commander par ordinateur, par exemple au moyen d'un automate décrit dans une autre demande de brevet de la demanderesse, déposée ce même jour.

On peut, de plus, réaliser non seulement la séparation des macromolécules dans les plaques de gel, mais également leur transfert sur des membranes appropriées placées le long d'une des grandes faces des plaques de gel.

On peut également créer, par une distribution uniforme de potentiels sur les plans d'électrodes régulièrement réparts par rapport à la pile de plaques, un champ électrique uniforme à travers les plaques de la pile.

En variante, le procédé consiste également à faire varier l'amplitude et/ou le sens du champ électrique à travers les plaques de la pile, par variation de la distribution des potentiels sur les plans d'électrodes.

Les variations dans le temps des différences de potentiel entre les plans d'électrodes peuvent être synchrones et égales entre elles, pour faire varier dans le temps l'amplitude du champ électrique sans modifier sa distribution dans l'espace, ou bien les différences de potentiel entre plans d'électrodes peuvent être modifiées localement, pour faire varier localement l'intensité du champ électrique.

Selon une autre variante, le procédé selon l'invention consiste encore à modifier, cycliquement ou non, les différences de potentiel entre les plans d'électrodes.

On peut, de cette façon, faire migrer les macromolécules par à-coups dans une direction souhaitée de migration.

Selon encore une autre variante de l'invention, le procédé consiste également à disposer côte à côte au moins deux piles de plaques, en les juxtaposant dans une direction parallèle ou perpendiculaire à la direction précitée de migration.

On peut ainsi traiter par des champs électriques différents les plaques de gel des différentes piles.

L'invention propose également un dispositif d'électrophorèse multiple pour la migration contrôlée de macromolécules dans des plaques de gel, comprenant une cuve destinée à recevoir les plaques de gel et un liquide d'électrophorèse, des moyens de support des plaques de gel qui sont maintenues parallèles et espacées les uns des autres en formant au moins une pile, une pluralité d'électrodes allongées parallèles entre elles et aux plaques et disposées le long de deux extrémités opposées de la pile de plaques dans deux plans perpendiculaires aux plaques, des moyens de liaison entre les électrodes d'un même plan, permettant de les maintenir à un même potentiel et des moyens d'application de potentiels électriques à ces électrodes pour créer entre ces deux plans un champ électrique de migration des macromolécules dans les plaques de gel, caractérisé en ce qu'il comprend :
- d'autres électrodes allongées parallèles aux électrodes précitées et disposées dans la cuve entre les extrémités de la pile de plaques, de sorte que toutes les électrodes sont situées aux intersections de deux séries de plans, les plans d'une première série étant perpendiculaires aux plaques et à une première direction de migration des macromolécules, les plans de la seconde série étant parallèles aux plaques,
- des moyens de liaison entre les électrodes contenues dans un même plan de la première série, permettant de les maintenir à un même potentiel électrique, et des moyens de liaison entre électrodes de plans différents de la première série, permettant de maintenir une différence de potentiel entre deux plans consécutifs de la première série,
- des moyens de liaison entre les électrodes d'un même plan de la seconde série, permettant de les maintenir à un même potentiel électrique, et des moyens de liaison entre électrodes de plans différents de la seconde série, permettant de maintenir une différence de potentiel entre deux plans consécutifs de la seconde série,
- et des moyens de commande pour faire varier la distribution des potentiels des électrodes desdits plans, pour créer successivement dans les plaques de gel et dans le liquide d'électrophorèse un premier champ électrique (E1) parallèle à la première direction de migration, puis un second champ électrique (E2) parallèle à une seconde direction de migration qui est perpendiculaire aux plaques de gel.

De préférence, les électrodes sont réparties régulièrement entre les extrémités des plaques de gel, ainsi qu'entre ces plaques.
de préference, les moyens de liaison entre deux électrodes consécutives d'un même plan sont du type à conduction électrique commandée, variable entre un état de conduction à résistance sensiblement nulle et au moins un état de conduction à résistance de valeur prédéterminée, non nulle.

Ces moyens de liaison peuvent notamment être des composants électroniques, tels que des transistors, des thyristors, etc..., qui sont sélectivement conducteurs ou bloqués.

Ces moyens de liaison peuvent être commandés individuellement ou par groupes, et de préférence par des moyens qui sont communs à toutes les électrodes situées dans un même groupe de plans parallèles.

Dans un mode de réalisation de l'invention, les moyens de liaison entre électrodes et les moyens de commande sont portés par au moins une plaque de matière diélectrique, sur laquelle sont fixées les électrodes par une de leurs extrémités.

Selon une variante de réalisation de l'invention, le support est destiné à recevoir au moins deux piles de plaques disposées côte à côte et juxtaposées dans au moins une direction parallèle à leurs plans, et une série d'électrodes du type précité, comprenant les moyens précités de commande et de liaison entre électrodes, est associée à chaque pile de plaques, pour créer dans les plaques des différentes piles des champs électriques qui sont à volonté identiques ou différents, liés ou indépendants les uns des autres.

Selon un mode de réalisation particulier de l'invention, les électrodes précitées sont intégrées à un panier mobile formant le support des plaques de gel.

En pratique, il suffit de réunir entre elles ces électrodes par des plaques, perforées ou non, ou des grilles de matériau diélectrique pour disposer d'un panier support, entre les électrodes duquel on peut disposer les plaques de gel, et que l'on va immerger dans la cuve et extraire de la cuve par la face horizontale supérieure de celle-ci.

Il est prévu également que les extrémités inférieures des électrodes précitées comportent des moyens de liaison ou de raccordement à des électrodes portées par la paroi de fond de ladite cuve.

Il suffit donc, dans ce cas, de déposer le panier sur le fond de la cuve pour que les électrodes verticales du panier puissent être reliées aux moyens d'application de potentiel et aux circuits de commande du dispositif, par l'intermédiaire des électrodes du fond de la cuve.

Avantageusement, ces moyens de liaison ou de raccordement forment également des moyens de positionnement du panier dans la cuve.

De préférence, les électrodes du fond de la cuve sont sensiblement ponctuelles, et forment un réseau à maille carrée ou rectangulaire, à côtés respectivement parallèles et perpendiculaires à la direction souhaitée de migration des macromolécules.

Elles sont reliées entre elles par des moyens de liaison commandés, permettant sélectivement de maintenir à un même potentiel les électrodes situées dans un même plan perpendiculaire à la direction souhaitée de migration des macromolécules, et d'établir une différence de potentiel entre deux successifs de ces plans.

L'invention prévoit également, dans ce cas, que les parois verticales de la cuve comportent des électrodes linéaires verticales, raccordées également à des moyens commandés de liaison permettant sélectivement d'établir entre elles des différences de potentiel, ou bien de les mettre à un même potentiel.

Afin d'augmenter la capacité de traitement du dispositif selon l'invention, le panier peut contenir, à un même niveau, au moins deux séries de plaques de gel, disposées tête-bêche, c'est-à-dire dans lesquelles les plaques d'une série sont sensiblement dans le prolongement des plaques de l'autre série, mais orientées longitudinalement dans un sens opposé, par rapport à la position initiale des macromolécules.

On peut ainsi traiter plusieurs doubles séries de plaques de gel, sans augmenter pour autant la différence de potentiel totale dans la cuve au-delà de celle requise pour une plaque.

De préférence, les électrodes verticales du panier sont entourées par des tubes cylindriques de matière diélectrique, perforée ou poreuse vis à vis du liquide d'électrophorèse.

Ces tubes, ouverts à leurs extrémités, forment des pièges pour les bulles de gaz produites par électrolyse au contact des électrodes, et guident les bulles vers la surface libre du liquide, sans qu'elles puissent venir au contact des plaques de gel.

Dans la description qui suit, faite à titre d'exemple, on se réfère aux dessins annexés, dans lesquels:
- la figure 1 représente schématiquement un appareil connu de séparation de macromolécules par électrophorèse ;
- la figure 2 est une vue schématique de bout d'une série d'électrodes propres à être utilisées dans un dispositif selon l'invention ;
- la figure 3 représente la même série d'électrodes, mais commandée de façon différente ;
- la figure 4 représente schématiquement une série semblable d'électrodes et ses moyens de commande ;
- la figure 5 représente schématiquement, en vue de dessus, la disposition de quatre séries d'électrodes selon l'invention, pour quatre piles de plaques de gel ;
- les figures 6 et 7 sont deux vues schématiques en perspective d'un réseau d'électrodes selon deux réalisations différentes de l'invention ;
- la figure 8 est une vue schématique en élévation d'un panier de support de plaques de gel ;
- la figure 9 est une vue de côté de ce panier ;
- la figure 10 est une vue schématique en perspective d'un panier de support de plaques ;
- la figure 11 est une vue schématique en perspective, avec arrachement partiel, d'une cuve d'électrophorèse;
- la figure 12 est une vue partielle, à plus grande échelle, d'un moyen de positionnement de panier dans la cuve ;
- la figure 13 est une vue schématique du circuit de liaison des électrodes du fond de la cuve ;
- la figure 14 est une vue schématique en perspective, avec arrachement partiel d'une autre forme de réalisation d'une cuve d'électrophorèse ;
- la figure 15 est une vue schématique partielle, en coupe transversale, de tubes poreux montés autour des électrodes verticales d'un panier.

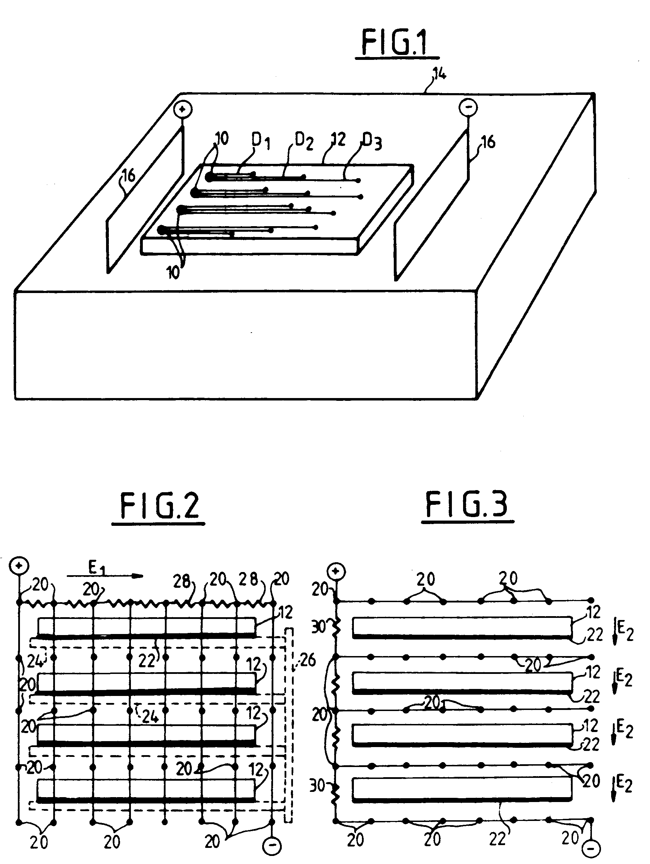

En figure 1, on a représenté très schématiquement le principe de la séparation des macromolécules dans une plaque de gel par électrophorèse.

Des échantillons contenant des macromolécules, par exemple des acides nucléiques, sont déposés dans des puits 10 formés dans une plaque rectangulaire 12 d'un gel d'agarose ou de polyacrylamide, le long d'un des petits côtés de cette plaque. La plaque de gel 12 contenant les échantillons est disposée dans une cuve 14 d'électrophorèse entre deux électrodes 16 (respectivement une anode et une cathode), dans la position représentée en figure 1. La cuve 14 est ensuite remplie d'un liquide approprié d'électrophorèse, puis les électrodes 16 sont reliées aux bornes d'une source d'énergie électrique, de façon à ce qu'il s'établisse entre les électrodes une différence prédéterminée de potentiel créant un champ électrique allant d'une électrode à l'autre dans la direction voulue de migration des macromolécules contenues dans les puits 10, à travers la plaque de gel 12.

Les vitesses de migration des macromolécules à travers la plaque 12, en direction du bord opposé de cette plaque, sont fonction de leur masse moléculaire. En conséquence, au bout d'un temps donné, les macromolécules auront parcouru dans la plaque 12 des distances qui sont fonction de leur masse moléculaire. On a représenté, à titre d'exemple, des distances D1, D2, D3 parcourues par des macromolécules depuis un puits 10.

Cette séparation des macromolécules en fonction de leur masse moleculaire permet, après transfert sur une membrane et marquage par hybridation ou autre procédé, d'identifier et de reconnaître les macromolécules marquées au moyen de sondes appropriées.

Les applications d'une telle technique sont multiples et intéressent de plus en plus l'industrie. Cependant, les appareils connus d'électrophorèse ne peuvent être utilisés qu'en laboratoire et ne sont pas susceptibles d'automatisation.

Il en est ainsi notamment parce que le milieu dans lequel s'établit le champ électrique, entre les électrodes 16, est particulièrement hétérogène : il comprend la plaque 12, la membrane de transfert qui se trouve le long d'une grande face de la plaque 12, le liquide d'électrophorèse qui baigne la plaque 12 et les électrodes 16, le support de la plaque 12 et de la membrane, etc... Le champ électrique entre les électrodes 16 n'est donc pas uniforme et n'est pas non plus orienté en tous points parallèlement à la direction souhaitée de migration des macromolécules dans la plaque 12. Par réaction électrochimique, il se produit également des bulles dans le liquide d'électrophorèse au contact des électrodes, en raison de la différence de potentiel relativement importante entre les électrodes 16 et de leur faible surface, et ces bulles qui se détachent des électrodes sont susceptibles de gêner ou d'influencer les migrations des macromolécules.

Comme indiqué, le procédé et le dispositif selon l'invention permettent d'éviter ces inconvénients de la technique actuelle et en outre d'automatiser la séparation et le transfert des macromolécules par électrophorèse.

Le procédé et le dispositif selon l'invention permettent également de traiter simultanément un grand nombre de plaques de gel, comprenant des échantillons de macromolécules à séparer.

On fera donc maintenant référence aux figures 2 et 3, qui illustrent schématiquement certaines caractéristiques essentielles de l'invention, applicables notamment à la migration des macromolécules à travers les plaques de gel, et au transfert des macromolécules sur les membranes associées aux plaques de gel.

Le dispositif selon l'invention comprend essentiellement, dans une cuve d'électrophorèse de dimensions appropriées, une série d'électrodes 20 de forme allongée, constituées par exemple par des fils de matériau électroconducteur approprié. Les électrodes 20 sont parallèles entre elles et s'étendent perpendiculairement au plan du dessin dans les figures 2 et 3. Elles sont disposées aux intersections de deux séries de plans perpendiculaires, dont les uns sont perpendiculaires à la direction souhaitée de migration des macromolécules, et dont les autres sont perpendiculaires aux premiers et à la direction de transfert des macromolécules sur les membranes.

Les électrodes 20 situées dans un même plan perpendiculaire à la direction souhaitée de migration, sont reliées entre elles de façon à se trouver au même potentiel, tandis que les électrodes situées dans des plans différents de ce type doivent être à des potentiels différents, pour l'établissement d'un champ électrique parallèle à la direction souhaitée de migration. Par ailleurs, les électrodes sont réunies entre elles, de la façon voulue, uniquement à leurs extrémités de façon à permettre la disposition, dans le réseau d'électrodes, d'une pile de plaques de gel 12 dont une grande face est pourvue d'une membrane de transfert 22 et qui sont portées par des supports 24 d'un panier 26, représenté en traits fantômes uniquement en figure 2. Les plaques de gel 12 forment ainsi un empilement vertical, en étant maintenues espacées les unes des autres et sont disposées de façon équidistante ou non, à volonté, entre des plans parallèles d'électrodes 20 en traversant chacune une série de plans perpendiculaires délimités par des électrodes 20.

Une électrode d'un plan vertical d'extrémité, par exemple l'électrode située au coin supérieur gauche de dessin de la figure 2, et l'électrode diagonalement opposée, sont reliées à deux bornes d'une source de tension électrique continue, les électrodes situées dans un même plan vertical sont reliées entre elles pour être au même potentiel, et la série d'électrodes située dans un même plan vertical est reliée à la série d'électrodes située dans un autre plan vertical adjacent ou consécutif par une résistance électrique 28 d'un pont diviseur de tension établissant entre elles de proche en proche une différence de potentiel prédéterminée.

Lorsqu'on souhaite obtenir un champ électrique uniforme à travers toutes les plaques 12, les résistances 28 ont toutes la même valeur et les plans verticaux d'électrodes sont équidistants.

Les plans verticaux parallèles délimités par les électrodes 20 sont des surfaces équipotentielles, au moins au niveau des électrodes 20 qu'ils contiennent. Le champ électrique E₁ ou gradient de potentiel développé entre les électrodes est normal aux surfaces équipotentielles et est donc, de par la disposition géométrique des électrodes et des plaques de gel 12, parallèle en un très grand nombre de points à la direction souhaitée de migrations des macromolécules, en ayant une amplitude sensiblement constante.

Il en résulte que les résultats de séparation des macromolécules par migration à travers les plaques de gel 12 sont, au moins en première approximation, fidèles et répétitifs. Un étalonnage ou calibrage préalable du dispositif permettra, si nécessaire, de déterminer avec précision les éventuelles singularités du champ électrique qui seront de toute façon faibles, et d'en tenir compte pour une appréciation des résultats de séparation.

Corollairement, le procédé et le dispositif selon l'invention permettent de traiter simultanément un très grand nombre de plaques de gel 12.

La même disposition d'électrodes 20 est utilisée en figure 3 pour le transfert des macromolécules sur les membranes 22 associées aux plaques 12. Seules changent, par rapport à la figure 2, les liaisons entre électrodes, puisque l'on veut obtenir un champ électrique perpendiculaire aux plans des plaques 12.

Pour cela, les électrodes 20 contenues dans un même plan horizontal, parallèle aux plaques 12, sont reliées pour être au même potentiel, tandis que la série d'électrodes contenue dans un même plan horizontal est reliée à la série d'électrodes contenue dans un autre plan horizontal adjacent ou consécutif par une résistance 30 de valeur déterminée. Les plans horizontaux contenant les électrodes 20 définissent ainsi des surfaces équipotentielles, auxquelles est perpendiculaire le champ électrique E₂ produit quand le plan horizontal supérieur d'électrodes 20 et le plan horizontal inférieur d'électrodes sont reliés à deux bornes opposées de la source de tension continue.

Lorsque toutes les résistances électriques 30 ont la même valeur, le champ électrique de transfert E₂ a la même amplitude pour toutes les plaques 12, quelle que soit leur disposition dans la pile.

Il est bien évident cependant qu'il suffit de modifier les valeurs des résistances 28 et 30 pour obtenir des distributions particulières de champ électrique à travers les plaques 12, dans la direction de migration et dans la direction de transfert des macromolécules. En contrôlant les valeurs des potentiels appliquées aux électrodes des plans d'extrémité, et la variation dans le temps de ces potentiels, on peut faire varier, localement ou dans l'ensemble du dispositif, l'amplitude du champ électrique, non seulement de façon permanente, mais également de façon cyclique. On peut ainsi, notamment, inverser la direction du champ électrique pendant une durée prédéterminée, puis l'inverser à nouveau pour le réorienter dans la direction souhaitée de migration ou de transfert.

On peut également réaliser un balayage, par commutation, d'une distribution donnée de potentiels appliqués à des plans successifs d'électrodes, cette distribution pouvant notamment comporter une inversion de champ électrique, que l'on fera se déplacer de proche en proche dans le réseau d'électrodes

On peut encore balayer les plans successifs d'électrodes ou certains d'entre eux par une différence de potentiel déterminée, par exemple pour la migration des macromolécules dans les plaques de gel. Cela permet de faire agir le champ électrique de façon répétitive sur des fractions déterminées de la longueur des plaques de gel, ou sur toute leur longueur si on le souhaite, en créant ce champ au moyen d'une différence de potentiel plusieurs fois inférieure à la différence de potentiel qu'il faudrait appliquer, pour obtenir un champ électrique de même intensité, aux électrodes situées aux extrémités opposées des plaques de gel. Il en résulte des avantages importants, au niveau de la consommation d'énergie, du choix des composants de commutation des potentiels, de l'échauffement du liquide, de la formation des bulles dans ce liquide, etc...

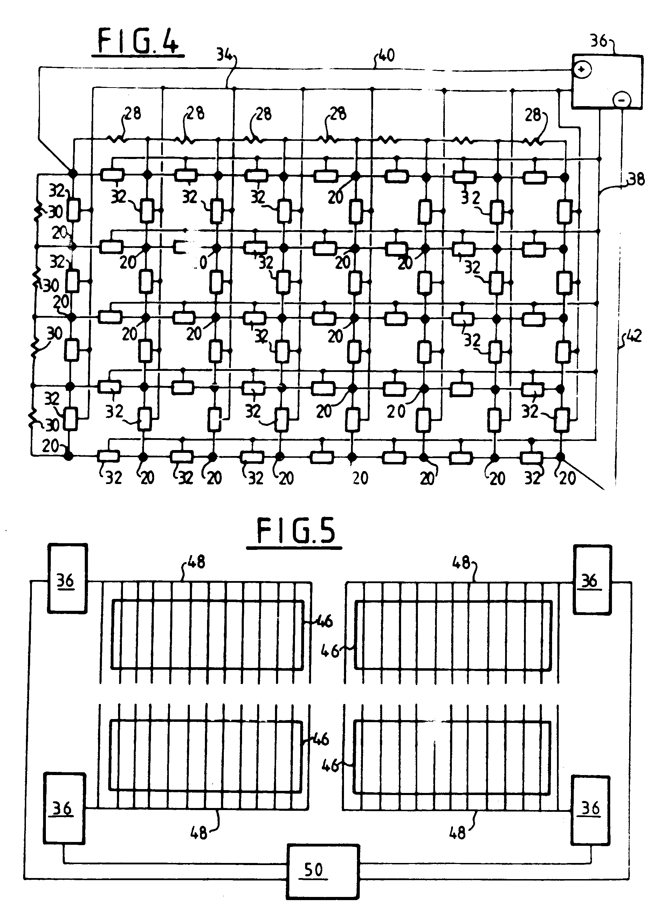

La figure 4 représente schématiquement, en vue de bout, la disposition d'électrodes et leurs moyens de liaison qui permettent d'obtenir notamment les agencements des figures 2 et 3 pour la migration et le transfert, respectivement, des macromolécules.

En figure 4, chaque électrode 20 est disposée selon l'intersection d'un plan horizontal et d'un plan vertical (tous deux perpendiculaires au plan du dessin) et est reliée aux électrodes voisines par un composant électronique 32 à conduction commandée, telle qu'un transistor ou un thyristor, qui peut être sélectivement conducteur et bloqué, c'est-à-dire dont la résistance électrique est soit sensiblement nulle, soit sensiblement infinie.

Dans le mode de réalisation représenté, les composants 32 reliant les électrodes 20 situées dans des plans horizontaux différents sont tous reliés, par leur entrée de commande, à une même ligne 34 aboutissant à un circuit de commande 36. De même, tous les composants 32 reliant entre elles des électrodes 20 situées dans des plans verticaux consécutifs, sont tous reliés, par leur entrée de commande, à une même ligne 38 aboutissant au circuit de commande 36. Ce circuit commande également les potentiels des électrodes 20 situées au coin supérieur gauche et au coin supérieur droit des dessins de la figure 4, par des lignes conductrices 40 et 42 respectivement.

Les électrodes contenues dans un même plan vertical sont reliées aux électrodes contenues dans le plan vertical adjacent par une résistance 28, et les électrodes contenues dans un plan horizontal sont reliées aux électrodes du plan horizontal adjacent par une résistance 30, ces résistances pouvant être de même valeur ou avoir des valeurs différentes, selon les cas de figure. On peut en particulier utiliser des résistances variables.

Lorsque tous les composants 32 reliant entre elles des électrodes situées dans des plans horizontaux sucessifs sont commandés pour avoir une résistance sensiblement nulle, et que tous les composants 32 reliant entre elles des élecrodes situées dans des plans verticaux successifs sont commandés pour avoir une résistance sensiblement infinie, on obtient l'agencement de la figure 2.

Inversement, lorsque les composants 32 reliant entre eux des plans horizontaux successifs sont commandés pour avoir une résistance sensiblement infinie, et que les composants 32 reliant entre eux des plans verticaux successifs sont commandés pour avoir une résistance sensiblement nulle, on obtient l'agencement de la figure 3.

Lorsque les résistances 28 et 30 sont remplacées par des sources de potentiel variable, dont la valeur est commandée par un circuit de commande approprié tel qu'un amplificateur opérationnel, on obtient des champs électriques qui sont localement différents d'une zone d'une plaque à une autre.

Au moyen du circuit de commande 36, on peut également faire varier de la façon souhaitée les potentiels appliqués par les lignes 40 et 42 aux électrodes des coins supérieur gauche et inférieur droit du réseau de la figure 4. On peut, de cette façon, inverser temporairement le champ électrique, à intervalles réguliers ou non, par exemple pour obtenir un champ électrique pulsé. On peut également balayer les plaques par une séquence de champs électriques qui sont localement différents, voire opposés.

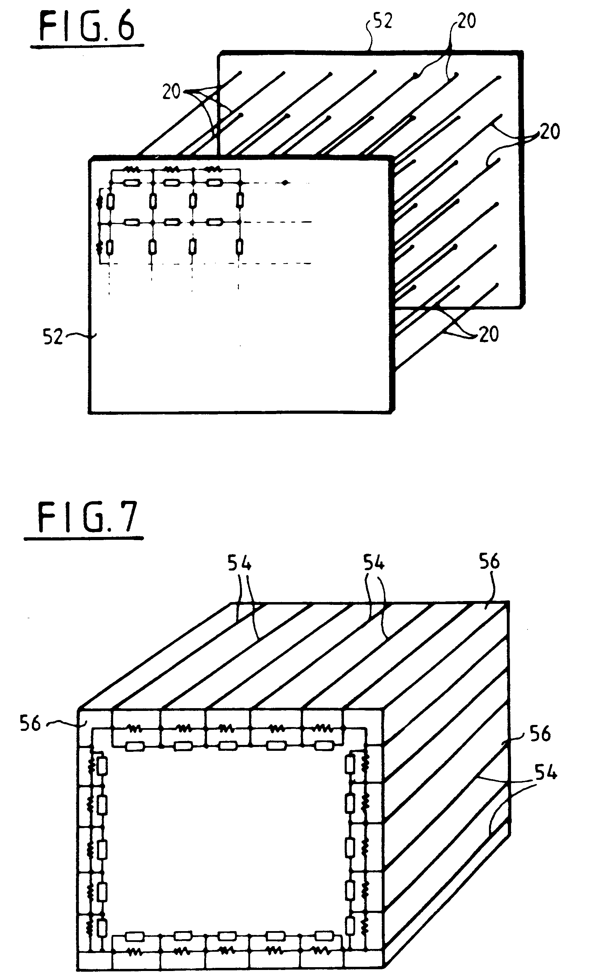

Lorsque des séries de plaques de gel doivent être soumises à des champs électriques différents, c'est-à-dire dont les distributions dans l'espace et/ou les variations dans le temps sont différentes, on peut utiliser l'agencement représenté en figure 5 qui permet le traitement simultané d'un certain nombre de piles 46 de plaques de gel. Chaque pile 46 est associée à un système 48 d'électrodes du même type que celui de la figure 4, comprenant un circuit 36 de commande des variations du champ électrique à travers la pile de plaques. Chaque circuit de commande 36 est relié lui-même à un système central de commande 50 qui est par exemple piloté par un ordinateur. Dans ce cas, les variations de potentiel dans les systèmes d'électrodes 48 associés aux différentes piles de plaques 46 peuvent être à volonté identiques ou différentes, liées les unes aux autres ou indépendantes les unes des autres.

Le procédé et le dispositif selon l'invention permettent donc de réaliser des séparations de macromolécules de masses moléculaires différentes dans des conditions uniformes, ou encore d'étudier le comportement de macromolécules de même nature et de même masse moléculaire dans des champs électriques différents et dans des plaques de gel de nature différente.

Un avantage de l'agencement d'électrodes selon l'invention est que des plaques de gel peuvent occuper tous les emplacements prévus entre les plans d'électrodes, ou seulement certains d'entre eux, sans qu'il en résulte une modification de la distribution des champs électriques.

En pratique (figure 6), les électrodes 20 seront toutes fixées, par une extrémité, sur une même plaque 52 de matériau diélectrique incluant les composants 28, 30, 32 et les liaisons nécessaires. Comme les électrodes seront avantageusement des fils de matériau conducteur, elles seront fixées à leur autre extrémité sur une seconde plaque de matériau diélectrique qui pourra comporter, ou non, certains des composants de liaison nécessaires.

La source de potentiel et les circuits de commande 36, 50 seront de préférence à l'extérieur du bain de liquide d'électrophorèse.

Comme on l'a représenté schématiquement en figure 2, les différentes plaques de gel sont montées sur des supports faisant partie d'un panier 26, déplaçable par translation à l'intérieur et à l'extérieur du réseau d'électrodes.

En variante (figure 7), les électrodes peuvent être limitées à des bandes conductrices 54 parallèles formées sur des parois 56 de la cuve, par exemple par métallisation.

Les cuves d'électrophonèse selon l'invention sont, de façon connue, équipées d'un système de circulation et de refroidissement du liquide d'électrolyse.

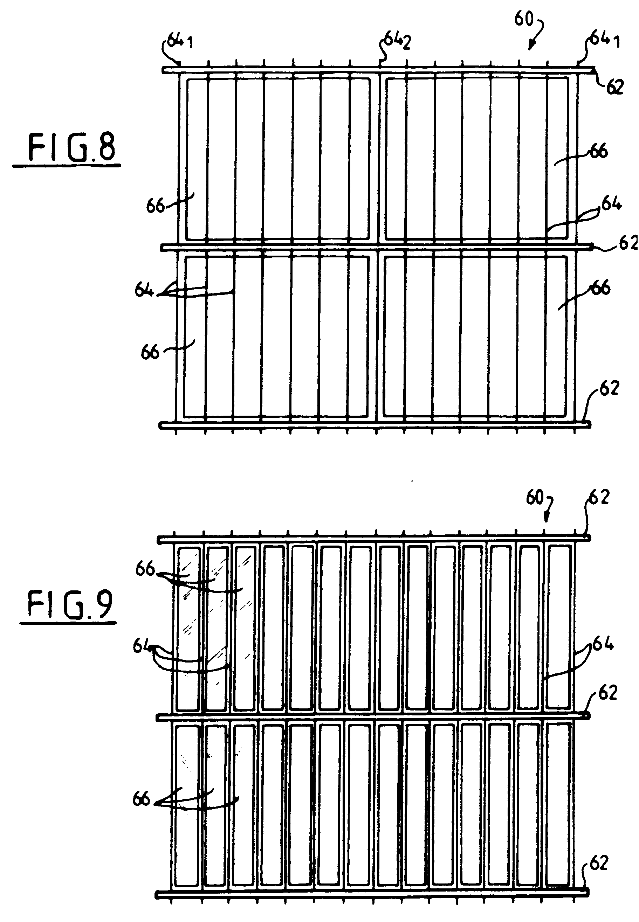

On se réfère maintenant aux figures 8 et 9 où l'on a représenté schématiquement un panier de support de plaques de gel selon l'invention.

Ce panier 60 comprend essentiellement des plaques horizontales 62 verticalement superposées et séparées les unes des autres d'une distance égale à la hauteur d'une plaque de gel disposée verticalement, certaines plaques 62 étant en retrait des parois de la cuve et/ou perforées pour permettre le passage des bulles de gaz, ou même constituées par des grilles.

Les plaques 62, en matériau diélectrique, sont reliées entre elles par des électrodes verticales rectilignes 64 qui peuvent être des tiges rigides de matériau électroconducteur, ou encore de simples fils électroconducteurs tendus entre les plaques 62, dans ce cas reliées par des montants rigides, non électriquement conducteurs, possédant la résistance mécanique voulue et disposés par exemple aux coins et au centre des plaques 62.

Les électrodes 64 sont disposées en un réseau à maille transversale rectangulaire ou carrée et ont essentiellement pour fonction d'être des lignes d'accrochage de surfaces équipotentielles.

Les plaques de gel 66 sont disposées verticalement sur les plaques 62, entre les électrodes 64, de telle sorte que la direction souhaitée de migration des macromolécules dans les plaques de gel soit horizontale.

Comme on le voit sur les figures 8 et 9, on peut disposer aux différents niveaux du panier 60 un assez grand nombre de plaques de gel 66, qui sont côte à côte et qui, à chaque niveau, forment par exemple deux séries de plaques, les plaques d'une série étant situées dans le prolongement des plaques correspondantes de l'autre série.

Comme on le souhaite, les plaques d'une série peuvent être orientées dans le même sens que les plaques de l'autre série, ou bien en sens contraire. Dans le premier cas, la différence de potentiel entre les extrémités du panier sera de l'ordre de deux fois la différence de potentiel à appliquer aux extrémités d'une plaque de gel. Dans le second cas, la différence de potentiel entre les extrémités du panier sera sensiblement égale à la différence de potentiel entre les extrémités d'une plaque de gel : on appliquera par exemple une tensions V1 aux électrodes 64₁ des extrémités du panier, et une tension V2 aux électrodes 64₂ du plan médian transversal du panier, avec la différence V1 - V2 égale à la différence de potentiel à appliquer entre les extrémités d'une plaque de gel.

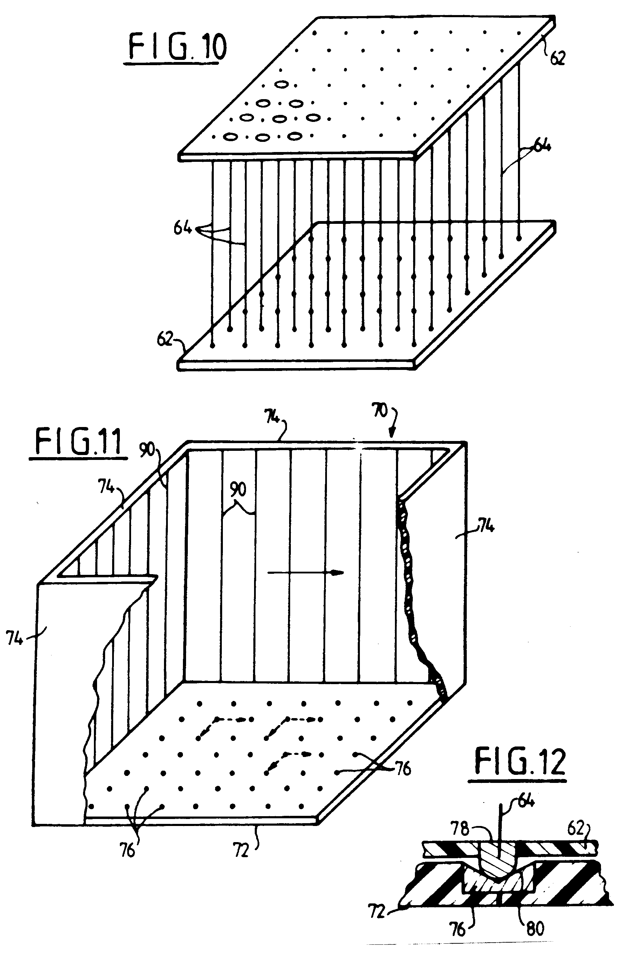

La figure 10 est une vue en perspective d'une variante de réalisation du panier, qui ne comprend qu'un seul niveau de chargement des plaques de gel, délimité entre deux plaques 62 de matériau diélectrique, dont seule la plaque supérieure est perforée pour permettre le passage des bulles de gaz formées par électrolyse dans le liquide d'électrophorèse.

La figure 11 représente schématiquement une cuve destinée à recevoir le panier de la figure 10.

Cette cuve 70 est de forme parallélépipédique et comprend un fond 72 horizontal et quatre parois verticales 74, avec éventuellement une paroi horizontale supérieure mobile, non représentée sur le dessin.

Le fond 72 de la cuve comprend une série d'électrodes sensiblement ponctuelles 76 formant un réseau à maille carrée ou rectangulaire, identique à celui des électrodes 64 du panier, de telle sorte que, lorsque le panier sera placé dans la cuve, les électrodes ponctuelles 76 du fond 62 de la cuve se trouvent exactement dans le prolongement des électrodes verticales 64 du panier.

Comme représenté en figure 12, les extrémités inférieures des électrodes 64 peuvent comprendre des moyens de contact avec les électrodes ponctuelles 76 et de positionnement du panier dans la cuve.

Dans l'exemple représenté, chaque électrode filiforme 64 du panier est raccordée, à son extrémité inférieure, à un plot conducteur 78 ayant une surface inférieure semi-sphérique en saillie sur le face inférieure de la plaque de fond 62 du panier, et qui est reçue dans un évidement tronconique 80 d'un plot conducteur formant une électrode ponctuelle 76 du fond de la cuve.

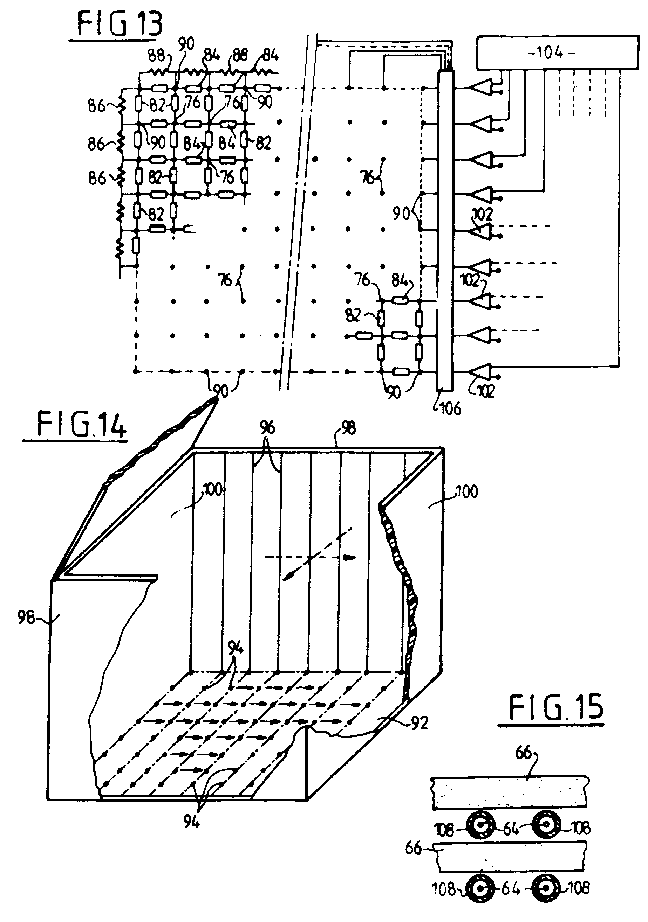

Les électrodes ponctuelles 76 sont reliées entre elles, à l'extérieur de la cuve, par le circuit représenté en figure 13 et déjà décrit en référence à la figure 4, qui comprend des moyens de liaison commandés entre électrodes 76 voisines. Les différentes rangées d'électrodes 76 sont reliées entre elles par des éléments de liaison 82 qui sont conducteurs avec une résistance électrique sensiblement nulle, ou bloqués avec une résistance électrique sensiblement infinie. De même, les différentes colonnes d'électrodes ponctuelles 76 sont reliées entre elles par des éléments de liaison 84, du même type que les éléments 82. En outre, des résistances 86, ou des éléments conducteurs équivalents, sont prévues entre les différentes rangées d'électrodes 76, et des résistances 88, ou des éléments conducteurs équivalents, sont prévues entre les différentes colonnes d'électrodes ponctuelles 76.

Les éléments de liaison 82 et 84 sont commandés de la façon suivante : quand les éléments de liaison 82 ont une résistance électrique sensiblement nulle, les éléments 84 ont une résistance électrique sensiblement infinie, de telle sorte que les colonnes d'électrodes ponctuelles 76 définissent des lignes équipotentielles, et que deux colonnes successives d'électrodes ont entre elles une différence de potentiel déterminée par la valeur de la résistance 88 correspondante, les électrodes de l'angle supérieur gauche et de l'angle inférieur droit du circuit étant reliées à des sources de potentiel appropriées. Alternativement, lorsque les éléments de liaison 82 ont une résistance électrique sensiblement infinie, et les éléments 84 ont une résistance sensiblement nulle, les rangées d'électrodes ponctuelles définissent des lignes équipotentielles et deux rangées successives ont entre elles une différence de potentiel déterminée par la valeur de la résistance 86 correspondante.

Les parois verticales 74 de la cuve 70 peuvent comprendre (figure 11) des électrodes linéaires verticales 90, formées par exemple par des lignes métallisées de leur surface interne, qui sont raccordées entre elles et aux rangées ou aux colonnes, respectivement, d'électrodes ponctuelles 76 du fond de la cuve par des éléments 82, 84.

On comprend le fonctionnement de la cuve 70 représentée en figure 11. Le panier chargé du nombre voulu de plaques de gel est placé dans la cuve et y est positionné automatiquement, grâce aux moyens de la figure 12, qui assurent en même temps la liaison électrique entre les électrodes ponctuelles 76 et les électrodes 64 du panier. Par commande des éléments de liaison 82 et 84, on assure tout d'abord la migration des macromolécules dans les plaques de gel, parallèlement à la longueur de ces plaques, puis, en inversant les rôles des éléments de liaison 82 et 84, la migration des macromolécules à travers l'épaisseur des plaques de gel et leur transfert sur les membranes associées. Les rangées, puis respectivement les colonnes d'électrodes ponctuelles 76, ainsi que les électrodes linéaires 90 des faces verticales de la cuve, définissent des surfaces équipotentielles planes permettant, comme déjà expliqué, des mesures fiables et rigoureuses.

En figure 11, la flèche en trait plein indique la direction dans laquelle les plaques de gel sont orientées, et les flèches en pointillés indiquent les directions du champ électrique, pour la migration et le transfert respectivement des macromolécules.

Le déplacement du panier dans la cuve 70 s'effectue par translation verticale, à travers la face supérieure ouverte de la cuve 70, par exemple au moyen d'un bras robot qui permet d'amener le panier dans une station de chargement-déchargement où l'on peut préparer un panier, pendant qu'un autre panier est en cours de traitement dans la cuve 70.

Dans une variante de réalisation plus simple représentée en figure 13, le fond 92 de la cuve comprend des électrodes ponctuelles 94, disposées selon un réseau carré à maille carrée et formant des rangées dans lesquelles elles sont raccordées entre elles par des conducteurs noyés dans le fond de la cuve. Ces rangées correspondent respectivement aux rangées d'électrodes 64 d'un panier et sont raccordées à leurs extrémités à des électrodes verticales 96 formées sur deux faces verticales opposées 98 de la cuve. Ces électrodes 94 et 96 définissent, avec les électrodes rectilignes 64 d'un panier, des plans équipotentiels qui sont perpendiculaires à la direction souhaitée de migration des macromolécules.

On procède donc de la façon suivante : le panier est placé dans la cuve de telle sorte que les plaques de gel qu'il contient s'étendent perpendiculairement aux plans équipotentiels définis par les électrodes linéaires 94 et 96. L'électrophorèse permet alors d'assurer la migration des macromolécules dans les plaques de gel, parallèlement à la longueur des plaques. On peut ensuite sortir le panier de la cuve, le faire tourner de 90° autour d'un axe vertical, puis de la replacer dans la cuve, de telle sorte que les plaques de gel seront parallèles aux plans équipotentiels définis par les électrodes linéaires 94 et 96. L'électrophorèse provoquera alors la migration des macromolécules dans l'épaisseur des plaques de gel, et leur transfert sur les membranes associées.

Les deux flèches en pointillé indiquent les orientations longitudinales des plaques de gel, dans les deux positions respectives de migration et de transfert des macromolécules, et les flèches en traits pleins correspondent à l'orientation du champ électrique entre les plans d'électrodes.

En variante, on peut prévoir deux cuves telles que celles de la figure 13, montées au voisinage immédiat l'une de l'autre, et réaliser dans l'une la migration des macromolécules sur la longueur des plaques de gel, et dans l'autre, le transfert des macromolécules sur les membranes associées.

Les deux autres faces verticales 100 de la cuve de la figure 13 sont dépourvues d'electrodes.

On peut utiliser, pour fixer les potentiels des différents plans d'électrodes, des amplificateurs opérationnels, commandés par un système de traitement de l'information. Il est alors très simple d'obtenir entre les plans d'électrodes des champs électriques variables dans le temps et dans l'espace, selon des lois cycliques ou non, d'utiliser des champs électriques de type impulsionnel, etc...

Par exemple, comme représenté sur la partie droite de la figure 12, des amplificateurs opérationnels 102 sont reliés chacun, à leur sortie, à une rangée d'électrodes 76. Une de leurs entrées est reliée à une sortie d'un circuit de commande 104, tandis que leur autre entrée est à la masse.

Lorsque les électrodes 76 forment un réseau carré, un commutateur multiple 106 permet d'appliquer les tensions de sortie des amplificateurs 102 soit aux rangées, soit aux colonnes d'électrodes 76.

En figure 14, on a représenté schématiquement des tubes ou manchons cylindriques 108 entourant les électrodes verticales 64 d'un panier. Ces tubes 108, ouverts à leurs extrémités, sont en matière diélectrique et sont soit perforés soit poreux, pour permettre le passage du liquide d'électrophorèse à travers leur paroi en direction des électrodes 64. Ces tubes forment ainsi des pièges à bulles, dans lesquels les bulles de gaz produites par électrolyse sont guidées vers la surface libre du liquide, sans venir au contact des plaques de gel. Ils servant aussi de protection des électrodes 64, et empêchent les plaques de gel 66 de venir au contact des électrodes. De plus, on peut alors utiliser des électrodes fromées en un métal conducteur moins coûteux que le platine. Si d'éventuels dépôts sont formés par électrolyse au contact des ces électrodes, les tubes 108 évitent que ces dépôts puissent passer dans le bain de liquide dans lequel se trouvent les plaques de gel.

Dans ce qui précède, on a décrit des formes de réalisation de l'invention applicables au cas où, les plaques de gel étant verticales, les deux directions de migration et de transfert des macromolécules sont horizontales. Bien entendu, l'invention couvre aussi le cas où, les plaques de gel étant verticales, la direction de migration des macromolécules dans les plaques est verticale, et leur directdion de transfert sur les membranes associées est horizontale. Il suffit pour cela d'utiliser des paniers dans lesquels les électrodes 64 sont horizontales.

## Revendications

1. Procédé d'électrophorèse multiple pour la migration contrôlée de macromolécules dans des plaques de gel, consistant à immerger des plaques de gel (12) dans une cuve contenant un liquide d'électrophorèse, ces plaques comprenant des macromolécules le long d'un de leurs bords et formant au moins une pile dans laquelle elles sont parallèles et espacées les unes des autres, et à appliquer des potentiels électriques à des électrodes allongées (20) parallèles entre elles et aux plaques de gel (12) immergées dans ledit liquide et disposées le long de deux extrémités opposées de la pile de plaques dans deux plans perpendiculaires aux plaques, les électrodes d'un même plan étant portées à un même potentiel, pour créer entre ces plans un champ électrique de migration des macromolécules dans les plaques de gel, caractérisé en ce que :
- ladite cuve comprend d'autres électrodes allongées (20) parallèles aux électrodes précitées et disposées entre les extrémités de la pile de plaques, de sorte que toutes les électrodes sont situées aux intersections de deux séries de plans, les plans d'une première série étant perpendiculaires aux plaques et à une première direction de migration des macromolécules, les plans de la seconde série étant parallèles aux plaques,
- et en ce que le procédé consiste à appliquer des potentiels électriques aux électrodes (20) pour créer dans les plaques de gel et dans le liquide d'électrophorèse, successivement un premier champ électrique (E1) dont la direction est en tous points sensiblement parallèle à la première direction de migration et ensuite un second champ électrique (E2) parallèle à une seconde direction de migration qui est perpendiculaire aux plaques de gel,
- le premier champ électrique (E1) étant créé par application de potentiels électriques différents à des plans d'électrodes différents de la première série, les électrodes (20) contenues dans un même plan de la première série étant à un même potentiel électrique,
- le second champ électrique (E2) étant créé par application de potentiels électriques différents à des plans d'électrodes différents de la seconde série, les électrodes (20) contenues dans un même plan de la seconde série étant à un même potentiel électrique.

2. Procédé selon la revendication 1, caractérisé en ce que le second champ électrique (E2) est utilisé pour transférer les macromolécules sur des membranes (22) placées sur les plaques de gel (12).

3. Procédé selon la revendication 1 ou 2, caractérisé en ce que les plans d'électrodes sont régulièrement répartis le long des plaques de gel (12) et entre les plaques de gel.

4. Procédé selon la revendication 3, caractérisé en ce que les potentiels électriques sont distribués uniformément sur les plans d'électrodes pour créer un champ électrique uniforme à travers les plaques de gel (12).

5. Procédé selon une des revendications 1 à 3, caractérisé en ce qu'il consiste également à faire varier la distribution des potentiels électriques sur les plans d'électrodes pour faire varier l'amplitude et/ou la direction du champ électrique à travers les plaques de gel (12).

6. Procédé selon la revendication 5, caractérisé en ce que les variations dans le temps des différences de potentiels électriques entre les plans d'électrodes sont synchrones et égales entre elles, pour faire varier dans le temps l'amplitude du champ électrique sans modifier sa distribution dans l'espace.

7. Procédé selon la revendication 5, caractérisé en ce qu'il consiste à faire varier localement les potentiels électriques appliqués à des plans d'électrodes pour faire varier localement l'intensité du champ électrique.

8. Procédé selon la revendication 5, caractérisé en ce qu'il consiste à modifier temporairement ou cycliquement les différences de potentiel électrique entre les plans d'électrodes, notamment pour réaliser un balayage des plans d'électrodes par une différence de potentiel.

9. Procédé selon une des revendications 1 à 8, caractérisé en ce qu'il consiste également à disposer au moins deux piles (46) de plaques de gel côte-à-côte dans la cuve en les juxtaposant dans une direction parallèle ou perpendiculaire à une direction souhaitée de migration.

10. Procédé selon la revendication 9, caractérisé en ce qu'il consiste à faire varier de façon différente les champs électriques à travers les plaques des différentes piles (46).

11. Dispositif d'électrophorèse multiple pour la migration contrôlée de macromolécules dans des plaques de gel, comprenant une cuve destinée à recevoir les plaques de gel (12) et un liquide d'électrophorèse, des moyens (24, 26) de support des plaques de gel (12) qui sont maintenues parallèles et espacées les uns des autres en formant au moins une pile, une pluralité d'électrodes allongées (20) parallèles entre elles et aux plaques (12) et disposées le long de deux extrémités opposées de la pile de plaques dans deux plans perpendiculaires aux plaques, des moyens de liaison entre les électrodes d'un même plan, permettant de les maintenir à un même potentiel, et des moyens (36) d'application de potentiels électriques à ces électrodes pour créer entre ces deux plans un champ électrique de migration des macromolécules dans les plaques de gel, caractérisé en ce qu'il comprend :
- d'autres électrodes allongées (20) parallèles aux électrodes précitées et disposées dans la cuve entre les extrémités de la pile de plaques, de sorte que toutes les électrodes sont situées aux intersections de deux séries de plans, les plans d'une première série étant perpendiculaires aux plaques et à une première direction de migration des macromolécules, les plans de la seconde série étant parallèles aux plaques,
- des moyens (32) de liaison entre les électrodes (20) contenues dans un même plan de la première série, permettant de les maintenir à un même potentiel électrique, et des moyens (28, 30) de liaison entre électrodes de plans différents de la première série, permettant de maintenir une différence de potentiel entre deux plans consécutifs de la première série,
- des moyens (32) de liaison entre les électrodes (20) d'un même plan de la seconde série, permettant de les maintenir à un même potentiel électrique, et des moyens (28, 30) de liaison entre électrodes de plans différents de la seconde série, permettant de maintenir une différence de potentiel entre deux plans consécutifs de la seconde série,
- et des moyens de commande pour faire varier la distribution des potentiels des électrodes desdits plans, pour créer successivement dans les plaques de gel et dans le liquide d'électrophorèse un premier champ électrique (E1) parallèle à la première direction de migration, puis un second champ électrique (E2) parallèle à une seconde direction de migration qui est perpendiculaire aux plaques de gel.

12. Dispositif selon la revendication 11, caractérisé en ce que chaque plaque de gel (12) est associée à une membrane (22) sur laquelle les macromolécules sont transférées sous l'effet du second champ électrique (E2).

13. Dispositif selon la revendication 11 ou 12, caractérisé en ce que les électrodes (20) sont réparties régulièrement le long des plaques de gel et entre les plaques de gel.

14. Dispositif selon une des revendications 11 à 13, caractérisé en ce que tes moyens (30) de liaison entre deux électrodes consécutives d'un même plan sont du type à conduction électrique commandée, variable entre un état de conduction à résistance sensiblement nulle et au moins un état de conduction à résistance de valeur prédéterminée non nulle.

15. Dispositif selon la revendication 14, caractérisé en ce que les moyens (30) de liaison précités sont commandés par des moyens (36) communs à toutes les électrodes situées dans une même série de plans parallèles.

16. Dispositif selon une des revendications 11 à 15, caractérisé en ce que les moyens (28, 30, 32) de liaison entre électrodes et les moyens de commande (36) sont portés par au moins une plaque de matière diélectrique sur laquelle sont fixées les étectrodes (20) par une de leurs extrémités.

17. Dispositif selon une des revendications 11 à 16, caractérisé en ce que chaque plaque de gel (12) se trouve dans la cuve entre deux groupes d'électrodes coplanaires.

18. Dispositif selon une des revendications 11 à 17, caractérisé en ce que les moyens de support sont destinés a recevoir au moins deux piles (46) de plaques de gel disposées côte-à-côte et juxtaposées dans au moins une direction parallèle aux plaques, et en ce qu'une série d'électrodes (48) du type précité, comprenant des moyens de commande et des moyens de liaison entre électrodes, est associée à chaque pile (46) de plaques pour créer dans les plaques de ces piles des champs électriques qui sont à volonté identiques ou différents, liés ou indépendants les uns des autres.

19. Dispositif selon une des revendications 11 à 18, caractérisé en ce que les moyens de commande précités sont automatisés et pilotés par ordinateur.

20. Dispositif selon une des revendications 11 à 19, caractérisé en ce que les plaques de gel (66) sont disposées verticalement dans la cuve (70) entre lesdites électrodes (64).

21. Dispositif selon la revendication 20, caractérisé en ce que les électrodes (64) sont des barreaux d'un panier mobile (60) formant les moyens de support des plaques de gel (66).

22. Dispositif selon la revendication 21, caractérisé en ce que les extrémités inférieures des électrodes (64) comportent des moyens de raccordement à des électrodes (76, 94) portées par une paroi (79, 92) de fond de la cuve.

23. Dispositif selon la revendication 22, caractérisé en ce que les moyens (70) de raccordement forment également des moyens de positionnement du panier (60) dans la cuve.

24. Dispositif selon la revendication 22 ou 23, caractérisé en ce que les électrodes (76) de la paroi de fond (72) de la cuve sont des étectrodes sensiblement ponctuelles.

25. Dispositif selon la revendication 22 ou 23, caractérisé en ce que les électrodes (94) de la paroi de fond (92) de la cuve sont sensiblement ponctuelles et reliées entre elles par des conducteurs parallèles perpendiculaires à la direction souhaitée de migration des macromolécules dans les plaques de gel.

26. Dispositif selon une des revendications 22 à 25, caractérisé en ce que des parois verticales (74, 98, 100) de la cuve comportent des électrodes linéaires (90, 96) perpendiculaires à la direction souhaitée de migration des macromolécules et raccordées à des moyens de liaison commandés permettant sélectivement d'établir entre elles des différences de potentiel ou de les mettre à un même potentiel.

27. Dispositif selon une des revendications 21 à 26, caractérisé en ce que le panier (60) contient au moins, à un même niveau, deux séries de plaques de gel (66) dans lesquelles les plaques d'une série sont sensiblement dans le prolongement des plaques de l'autre série et sont orientées soit dans le même sens, soit dans le sens contraire.

28. Dispositif selon une des revendications 11 à 27, caractérisé en ce que les électrodes (64) sont entourées par des tubes cylindriques (108) en matière diélectrique, perforée ou poreuse, formant des pièges à bulles.

## Claims

1. Multiple electrophoresis method for the controlled migration of macromolecules in gel plates, consisting in immersing gel plates (12) in a tank containing an electrophoresis liquid, said plates including macromolecules along the length of one of their edges and forming at least one stack in which they are spaced apart from and parallel to one another, and in applying potentials to elongate electrodes (20) parallel to one another and to the gel plates (12) immersed in said liquid and disposed along two opposite ends of the stack of plates in two planes perpendicular to the plates, electrodes in the same plane being put at the same potential in order to create between said planes an electric field for causing the macromolecules in the gel plates to migrate, characterized in that:
- said tank includes other elongate electrodes (20) parallel to the above-mentioned electrodes and disposed between the ends of the stack of plates, such that all the electrodes are situated at the intersections of two series of planes, the planes of a first series being perpendicular to the plates and to a first direction of macromolecule migration, and the planes of the second series being parallel to the plates;
- and in that the method consists in applying potentials to the electrodes (20) in order to create successively in the gel plates and in the electrophoresis liquid: a first electric field (E1) whose direction is at all points substantially parallel to the first direction of migration and then a second electric field (E2) parallel to a second direction of migration that is perpendicular to the gel plates;
- the first electric field (E1) being created by application of different potentials to different electrode planes of the first series, electrodes (20) contained in the same first plane being at the same potential;
- the second electric field (E2) being created by applying different potentials to different electrode planes of the second series, electrodes (20) contained in the same plane of the second series being at the same potential.

2. Method according to claim 1, characterized in that the second electric field (E2) is used to transfer the macromolecules onto membranes (22) placed on the gel plates (12).

3. Method according to claim 1 or 2, characterized in that the electrode planes are evenly spaced apart along the length of the gel plates (12), and between the gel plates.

4. Method according to claim 3, characterized by an uniform distribution of potentials over the electrode planes, creating a uniform electric field through the gel plates (12).

5. Method according to any one of claims 1 to 3, characterized in that it consists in varying the amplitude and/or direction of the electric field through the gel plates (12) of the stack, by varying the potential distribution in the electrode planes.

6. Method according to claim 5 , characterized in that the variations in time of the potential differences between the electrode planes are synchronous and equal to each other, so as to cause the amplitude of the electric field to vary in time without modifying its distribution in space.

7. Method according to claim 5, characterized in that it consists in locally varying the potential applied to electrode planes, so as to cause the intensity of the electric field to vary locally.

8. Method according to claim 5 , characterized in that it consists in temporarily or cyclically modifying the potential differences between the electrode planes, for example so as to sweep the electrode planes with a potential difference.

9. Method according to any one of claims 1 to 8, characterized in that it consists in disposing at least two stacks (46) of gel plates side by side in the tank, while juxtaposing them in a direction parallel or perpendicular to the desired migration direction.

10. Method according to claim 9, characterized in that it consists in varying differently the electric fields through the plates of the different stacks (46).

11. Multiple electrophoresis device for the controlled migration of macromolecules in gel plates, the device comprising a tank receiving the gel plates (12) and an electrophoresis liquid, support means (24, 26) for supporting the gel plates (12) which are held spaced apart from and parallel to one another forming at least one stack, a plurality of elongate electrodes (20) parallel to one another and to the plates (12) and disposed along two opposite ends of the stack of plates in two planes perpendicular to the plates, connection means between the electrodes in the same plane for maintaining them at the same potential, and means (36) for applying potentials to said electrodes in order to create between said two planes an electric field for causing the macromolecules in the gel plates to migrate, the device being characterized in that it comprises:
- other elongate electrodes (20) parallel to the above-mentioned electrodes and disposed in the tank between the ends of the stack of plates, such that all the electrodes are situated at the intersections of two series of planes, the planes of a first series being perpendicular to the plates and to a first direction of macromolecules migration, and the planes of the second series being parallel to the plates.
- connection means (32) between electrodes (20) contained in the same plane of the first series, for maintaining the electrodes at the same potential, and connection means (28, 30) between electrodes of different planes of the first series, for maintaining a potential difference between two consecutive planes of the first series;
- connection means (32) between electrodes of the same plane of the second series, for maintaining the electrodes at the same potential, and connection means (28, 30) between electrodes of different planes of the second series, for maintaining a potential difference between two consecutive planes of the second series; and
- control means for causing the distribution of the potentials of the electrodes of said planes to vary, in order to create successively in the gel plates and in the electrophoresis liquid: a first electric field (E1) parallel to the first direction of migration ; and then a second electric field (E2) parallel to a second direction of migration that is perpendicular to the gel plates.

12. Device according to claim 11, characterized in that each gel plate (12) is associated with a membrane (22) onto which the macromolecules are transferred by the second electric field (E2).

13. Device according to any one of claims 11 or 12, characterized in that the electrodes (20) are spaced apart evenly along the gel plates, as well as between the gel plates.

14. Device according to any one of claims 11 to 13, characterized in that the connection means (30) between two consecutive electrodes of the same plane are of the controlled electric conduction type, variable between a conduction state with substantially zero resistance and at least a conduction state with resistance of a predetermined non zero value.

15. Device according to claim 14, characterized in that said connection means (30) are controlled by means (36) which are common to all the electrodes situated in the same group of parallel planes.

16. Device according to any one of claims 11 to 15, characterized in that means (28, 30, 32) for connection between electrodes and the control means (36) are carried by at least one plate of dielectric material, on which the electrodes (20) are fixed by one of their ends.

17. Device according to any one of claims 11 to 16, characterized in that each gel plate (12) is disposed in the electrophoresis tank between two groups of coplanar electrodes.

18. Device according to any one of claims 11 to 17, characterized in that the support means are intended to receive at least two stacks (46) of gel plates disposed side by side and juxtaposed in at least one direction parallel to the plates and in that a series of electrodes (48) of the above type comprising said control means and connection means between electrodes is associated with each stack (46) plates for creating in the plates of the different stacks electric fields which are identical or different, as desired, linked to or independent of each other.

19. Device according to any one of claims 11 to 18, characterized in that said control means are automated, and driven by computer.

20. Device according to any one of claims 11 to 19, characterized in that the gel plates (66) are disposed vertically in the tank (70) between said electrodes (64).

21. Device according to claim 20, characterized in that the electrodes (64) are bars of a mobile basket (60) forming the support for the gel plates (66).

22. Device according to claim 21, characterized in that the ends, for example the lower ends, of the electrodes (64) comprise means (78) for coupling to electrodes (76, 94) carried by the bottom wall (79, 92) of the tank.

23. Device according to claim 22, characterized in that the coupling means (70) also form means for positioning the basket (60) in the tank.

24. Device according to claim 22 or 23 characterized in that the electrodes (76) of the bottom wall (72) of the tank are substantially pinpoint electrodes.

25. Device according to claim 22 or 23, characterized in that the electrodes (94) of the bottom (92) of the tank are substantially pinpoint and connected together by parallel conductors perpendicular to the desired direction of migration of the macromolecules in the gel plates.

26. Device according to any one of claims 22 to 25, characterized in that the vertical walls (74, 98, 100) of the tank comprise linear electrodes (90, 96) perpendicular to the desired direction of migration of the macromolecules and connected to controlled connecting means for selectively providing potential differences therebetween or else placing them at the same potential.

27. Device according to any one of claims 21 to 26, characterized in that said basket (60) contains, at the same level, at least two series of gel plates (66) in which the plates of one series are substantially in the extension of the plates of the other series, and oriented either in the same direction or in an opposite direction.

28. Device according to any one of claims 11 to 27, characterized in that the electrodes (64) are surrounded by cylindrical tubes (108) of a dielectric material, which are perfored or porous and form bubble traps.

## Patentansprüche

1. Verfahren zur multiplen Elektrophorese zur kontrollierten Migration von Makromolekülen in Gelplatten, welches aufweist: das Eintauchen der Gelplatten (12) in einen Behälter mit einer elektrophoretischen Flüssigkeit, wobei diese Platten entlang eines Randes Makromoleküle enthalten und zumindest eine Zelle bilden, in welcher sie parallel und in Abstand voneinander angeordnet sind, und das Anlegen elektrischer Potentiale an langgestreckte Elektroden (20), die zueinander und zu den in der Flüssigkeit eingetauchten Gelplatten (12) parallel sind und entlang zwei gegenüberliegender Enden der Plattenzelle in zwei normal auf die Platten stehenden Ebenen angeordnet sind, wobei die in einer Ebene liegenden Elektroden auf gleichem Potential liegen, um zwischen diesen Ebenen ein elektrisches Feld zur Migration von Makromolekülen in den Gelplatten zu erzeugen, **dadurch gekennzeichnet, daß**
- dieser Behälter weitere langgestreckte Elektroden (20) aufweist, welche zu den oben genannten Elektroden parallel und so zwischen den Enden der Plattenzelle angeordnet sind, daß alle Elektroden an den Schnittlinien zweier Reihen von Ebenen angeordnet sind, wobei die Ebenen einer ersten Reihe normal auf die Platten und eine erste Migrationsrichtung stehen und die Ebenen der zweiten Reihe parallel zu den Platten sind,
- und daß das Verfahren das Anlegen elektrischer Potentiale an die Elektroden (20) aufweist, um in den Gelplatten und in der elektrophoretischen Flüssigkeit zuerst ein erstes elektrisches Feld (E1), das in allen Punkten im wesentlichen parallel zu der ersten Migrationsrichtung ausgerichtet ist, und dann ein zweites elektrisches Feld (E2) zu erzeugen, welches parallel zu einer zweiten Migrationsrichtung und normal auf die Gelplatten ausgerichtet ist,
- wobei das erste elektrische Feld (E1) durch Anlegen unterschiedlicher elektrischer Potentiale an unterschiedliche Elektrodenebenen der ersten Reihe erzeugt wird und die in einer Ebene der ersten Reihe enthaltenen Elektroden (20) auf gleichem elektrischen Potential liegen,
- und das zweite elektrische Feld (E2) durch Anlegen unterschiedlicher elektrischer Potentiale an unterschiedlichen Elektrodenebenen der zweiten Reihe erzeugt wird und die in einer Ebene der zweiten Reihe enthaltenen Elektroden (20) auf gleichem elektrischen Potential liegen.

2. Verfahren nach Anspruch 1, **dadurch gekennzeichnet, daß** das zweite elektrische Feld (E2) zum Überführen der Makromoleküle auf Membranen (22) verwendet wird, die auf den Gelplatten angeordnet sind.

3. Verfahren nach Anspruch 1 oder 2, **dadurch gekennzeichnet, daß** die Ebenen der Elektroden entlang der Gelplatten (12) und zwischen diesen Gelplatten in regelmäßigen Abständen angeordnet sind.

4. Verfahren nach Anspruch 3, **dadurch gekennzeichnet, daß** die elektrischen Potentiale gleichmäßig auf die Elektrodenebenen aufgeteilt sind, um in den Gelplatten (12) ein gleichmäßiges elektrisches Feld zu erzeugen.

5. Verfahren nach einem der Ansprüche 1 bis 3, **dadurch gekennzeichnet, daß** die Potentialaufteilung auf die Elektrodenebenen verändert wird, um die Amplitude und/oder die Richtung des elektrischen Feldes in den Gelplatten (12) zu verändern.

6. Verfahren nach Anspruch 5, **dadurch gekennzeichnet, daß** die zeitlichen Änderungen der elektrischen Potentialdifferenzen zwischen den Elektrodenebenen synchron erfolgen, um eine zeitliche Änderung der Amplitude des elektrischen Feldes herbeizuführen ohne seine Verteilung im Raum zu verändern.

7. Verfahren nach Anspruch 5, **dadurch gekennzeichnet, daß** es ein örtliches Verändern der an die Elektrodenebenen angelegten elektrischen Potentiale aufweist, um die Intensität des elektrischen Feldes örtlich zu verändern.

8. Verfahren nach Anspruch 5, **dadurch gekennzeichnet, daß** es ein zeitlich vorübergehendes oder zyklisches Verändern der elektrischen Potentialdifferenzen zwischen den Elektrodenebenen aufweist, um insbesondere ein Abtasten der Elektrodenebenen durch eine Potentialdifferenz zu ermöglichen.

9. Verfahren nach einem der Ansprüche 1 bis 8, **dadurch gekennzeichnet, daß** zumindest zwei Zellen (46) mit Gelplatten Seite-an-Seite in dem Behälter angeordnet und in einer Richtung parallel oder normal zu der gewünschten Migrationsrichtung miteinander verbunden sind.

10. Verfahren nach Anspruch 9, **dadurch gekennzeichnet, daß** die elektrischen Felder in den Platten der unterschiedlichen Zellen (46) in unterschiedlicher Weise verändert werden.

11. Vorrichtung zur multiplen Elektrophorese zur kontrollierte Migration von Makromolekülen in Gelplatten, die aufweist: einen Behälter, in welchem die Gelplatten (12) aufgenommen werden können, Mittel (24, 26) zur Halterung der Gelplatten (12), welche parallel und in Abstand voneinander gehalten werden und zumindest eine Zelle bilden, eine Mehrzahl von langgestreckten Elektroden (20), die zueinander und zu den Platten (12) parallel sind und entlang zweier gegenüberliegender Enden der Plattenzelle in zwei Ebenen normal auf die Platten angeordnet sind, Mittel zum Verbinden der Elektroden einer Ebene, durch welche sie auf gleichem Potential gehalten werden, und Mittel (36) zum Anlegen elektrischer Potentiale an diese Elektroden, um zwischen diesen beiden Ebenen in den Gelplatten ein elektrisches Feld zur Migration von Makromolekülen zu erzeugen, **dadurch gekennzeichnet, daß** sie aufweist:
- weitere langgestreckte Elektroden (20), die parallel zu den oben genannten Elektroden und so zwischen den Enden der Plattenzelle in dem Behälter angeordnet sind, daß alle Elektroden an den Schnittlinien von zwei Reihen von Ebenen liegen, wobei die Ebenen einer ersten Reihe normal auf die Platten und eine erste Migrationsrichtung der Makromoleküle stehen und die Ebenen der zweiten Reihe parallel zu den Platten sind,
- Mittel (32) zum Verbinden der Elektroden (20) einer Ebene der ersten Reihe, welche sie auf einem gemeinsamen elektrischen Potential halten, und Mittel (28, 30) zum Verbinden der Elektroden unterschiedlicher Ebenen der ersten Reihe, welche das Herstellen einer Potentialdifferenz zwischen zwei aufeinanderfolgenden Ebenen der ersten Reihe ermöglichen,
- Mittel (32) zum Verbinden der Elektroden (20) einer Ebene der zweiten Reihe, welche sie auf einem gemeinsamen elektrischen Potential halten, und Mittel (28, 30) zum Verbinden der Elektroden unterschiedlicher Ebenen der zweiten Reihe, welche das Herstellen einer Potentialdifferenz zwischen zwei aufeinanderfolgenden Ebenen der zweiten Reihe ermöglichen,
- Steuermittel zum Verändern der Potentialverteilung an den Elektroden der Ebenen, um in den Gelplatten und in der elektrophoretischen Flüssigkeit zuerst ein erstes elektrisches Feld (E1), welches parallel zu der ersten Migrationsrichtung ausgerichtet ist, und danach ein zweites elektrisches Feld (E2) zu erzeugen, welches parallel zu einer zweiten Migrationsrichtung ist, die Normal auf die Gelplatten ausgerichtet ist.

12. Vorrichtung nach Anspruch 11, **dadurch gekennzeichnet, daß** jede Gelplatte (12) einer Membran (22) zugeordnet ist, auf welche die Makromoleküle unter der Wirkung des zweiten elektrischen Feldes (E2) übertragen werden.

13. Vorrichtung nach Anspruch 11 oder 12, **dadurch gekennzeichnet, daß** die Elektroden (20) entlang der Gelplatten und zwischen diesen Gelplatten in regelmäßigen Abständen angeordnet sind.

14. Vorrichtung nach einen der Ansprüche 11 bis 13, **dadurch gekennzeichnet, daß** die Mittel (30) zum Verbinden zwei aufeinanderfolgender Elektroden einer Ebene vom Typ mit steuerbarer Leitfähigkeit sind, die von einem im wesentlichen widerstandslosen leitfähigen Zustand auf einen leitfähigen Zustand mit einem bestimmten Widerstand größer null veränderbar ist.

15. Vorrichtung nach Anspruch 14, **dadurch gekennzeichnet, daß** die Mittel (30) zum Verbinden durch Mittel (36) gesteuert werden, welche allen Elektroden gemeinsam sind, die in ein und derselben Reihe paralleler Ebenen liegen.

16. Vorrichtung nach einem der Ansprüche 11 bis 15, **dadurch gekennzeichnet, daß** die Mittel (28, 30, 32) zum Verbinden der Elektroden und die Steuermittel (36) von zumindest einer dielektrischen Platte gehalten werden, an welcher die Elektroden (20) mit ihren Enden befestigt sind.

17. Vorrichtung nach einem der Ansprüche 11 bis 16, **dadurch gekennzeichnet, daß** sich jede Gelplatte (12) in einem Behälter zwischen zwei Gruppen koplanarer Elektroden befindet.

18. Vorrichtung nach einem der Ansprüche 11 bis 17, **dadurch gekennzeichnet, daß** die Haltemittel zum Aufnehmen zumindest zweier Zellen (46) mit Gelplatten geeignet sind, welche Seite-an-Seite angeordnet und zumindest in einer Richtung parallel zu den Platten verbunden sind, und daß eine Elektrodenreihe (48) der vorhin genannten Art, welche Mittel zum Steuern und Mittel zum Verbinden von Elektroden aufweist, jeder Plattenzelle (46) zugeordnet ist, um in den Platten dieser Zellen elektrische Felder zu erzeugen, die gewünschtenfalls gleich oder unterschiedlich, miteinander verbunden oder voneinander unabhängig sind.

19. Vorrichtung nach einem der Ansprüche 11 bis 18, **dadurch gekennzeichnet, daß** die Mittel zu Steuern mittels eines Computers automatisier- und steuerbar sind.

20. Vorrichtung nach einem der Ansprüche 11 bis 19, **dadurch gekennzeichnet, daß** die Gelplatten (66) in dem Behälter (70) vertikal zwischen den Elektroden (64) angeordnet sind.

21. Vorrichtung nach Anspruch 20, **dadurch gekennzeichnet, daß** die Elektroden (64) Stäbe eines beweglichen Gitters (60) sind, welches die Halterungsmittel für die Gelplatten (66) bildet.

22. Vorrichtung nach Anspruch 21, **dadurch gekennzeichnet, daß** die unteren Enden der Elektroden (64) Mittel zum Anschließen dieser Elektroden (76, 94) aufweisen, welche von einer Wand (79, 92) am Boden des Behälters gehalten sind.

23. Vorrichtung nach Anspruch 22, **dadurch gekennzeichnet, daß** die Mittel (70) zum Anschließen ebenso Mittel zum Positionieren des Gitters (60) in dem Behälter bilden.

24. Vorrichtung nach Anspruch 22 oder 23, **dadurch gekennzeichnet, daß** die Elektroden (76) der Bodenfläche (72) des Behälters im wesentlichen punktförmig sind.

25. Vorrichtung nach Anspruch 22 oder 23, **dadurch gekennzeichnet, daß** die Elektroden (94) der Bodenfläche (92) des Behälters im wesentlichen punktförmig sind und untereinander mittels paralleler Leiter verbunden sind, welche normal zu der gewünschten Migrationsrichtung der Makromoleküle in den Gelplatten angeordnet sind.

26. Vorrichtung nach einem der Ansprüche 22 bis 25, **dadurch gekennzeichnet, daß** die Vertikalwände (74, 98, 100) des Behälters lineare Elektroden (90, 96) aufweisen, welche normal zu der gewünschten Migrationsrichtung der Makromoleküle stehen und an gesteuerte Mittel zum Verbinden angeschlossen sind, die es ermöglichen, zwischen diesen selektiv Potentialdifferenzen herzustellen oder sie auf gleichem Potential zu halten.

27. Vorrichtung nach einem der Ansprüche 21 bis 26, **dadurch gekennzeichnet, daß** das Gitter (60) zumindest auf einem Niveau zwei Reihen von Gelplatten (66) aufweist, bei welchen die Platten einer Reihe im wesentlichen in Verlängerung der Platten der anderen Reihe angeordnet und entweder gleich- oder gegensinnig ausgerichtet sind.

28. Vorrichtung nach einem der Ansprüche 11 bis 27, **dadurch gekennzeichnet, daß** die Elektroden (64) von zylindrischen Röhren (108) aus dielektrischem Material umgeben sind, welches löchrig oder porös ist und einen Getter für Blasen bildet.
